# EUROPEAN PATENT SPECIFICATION

(11) **EP 1 442 055 B1**
(45) Date of publication and mention of the grant of the patent: **02.07.2008**
(21) Application number: 02782941.5
(22) Date of filing: 17.10.2002
(51) Int. Cl.: C07K 14/435, G01N 33/53

(54) **AEQUORIN AS A GROWTH MARKER IN YEAST**
AEQUORIN ALS WACHSTUMSMARKER IN HEFE
AEQUORINE SERVANT DE MARQUEUR DE CROISSANCE DANS LA LEVURE

(30) Priority: 27.10.2001 EP 01125708
(43) Date of publication of application: 04.08.2004
(73) Proprietor: Sanofi-Aventis Deutschland GmbH, 65929 Frankfurt am Main (DE)
(72) Inventor: FRAISSIGNES, Pauline, F-76000 Roven (FR); GUEDIN, Denis, F-94420 Le Plessis Trevise (FR)
(86) International application number: PCT/EP2002/011631
(87) International publication number: WO 2003/037923

(56) References cited:
- US-A- 5 876 951
- PARNOT C ET AL: "SYSTEMATIC IDENTIFICATION OF MUTATIONS THAT CONSTITUTIVELY ACTIVATE THE ANGIOTENSIN II TYPE 1A RECEPTOR BY SCREENING A RONADOMLY MUTATED CDNA LIBRARY WITH AN ORIGINAL PHARMACOLOGICAL BIOASSAY" PROCEEDINGS OF THE NATIONAL ACADEMY OF SCIENCES OF USA, NATIONAL ACADEMY OF SCIENCE. WASHINGTON, US, vol. 97, no. 13, 30 June 2000 (2000-06-30), pages 7615-7620, XP001080457 ISSN: 0027-8424
- NAKAJIMA-SHIMADA J ET AL: "MONITORING OF INTRACELLULAR CALCIUM IN SACCHAROMYCES CEREVISIAE WITH AN APOAEQUORIN CDNA EXPRESSION SYSTEM" PROCEEDINGS OF THE NATIONAL ACADEMY OF SCIENCES OF USA, NATIONAL ACADEMY OF SCIENCE. WASHINGTON, US, vol. 88, no. 15, August 1991 (1991-08), pages 6878-6882, XP001026232 ISSN: 0027-8424
- NAKAJIMA-SHIMADA J ET AL.: "Ca2+ signal is generated only once in the mating pheromone response pathway in Saccharomyces cerevisiae." CELL STRUCT. FUNCT., vol. 25, no. 2, April 2000 (2000-04), pages 125-131, XP002191457
- TANAHASHI H. ET AL: 'Photoprotein aequorin: use as a reporter enzyme in studying gne expression in mammalina cells.' GENE vol. 96, 1990, pages 249 - 255

## Description

The present invention relates to a modified yeast cell containing aequorin as a growth marker - methods of making and methods of using such modified yeast cells.

Aequorin is a photoprotein isolated from luminescent jellyfish Aequoria victoria.

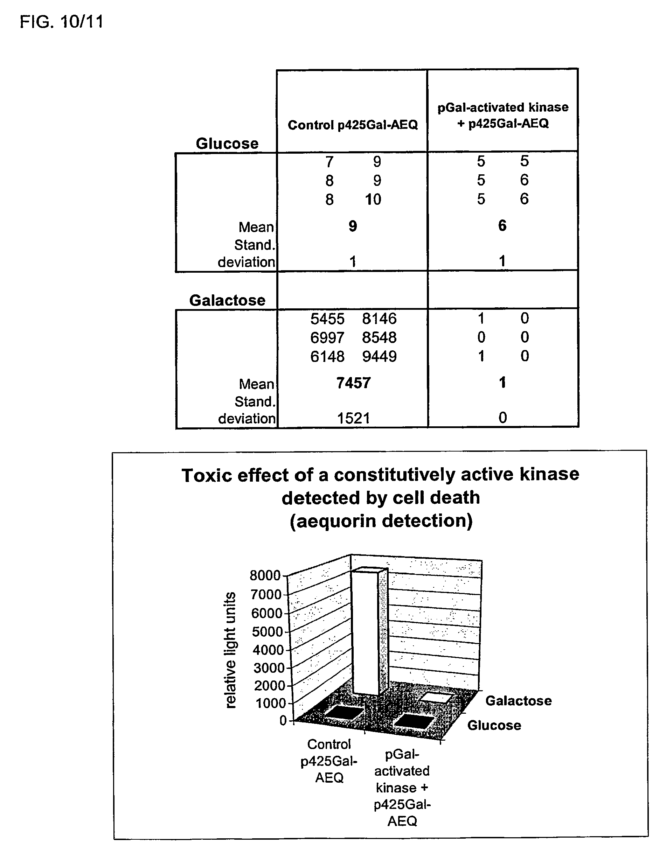

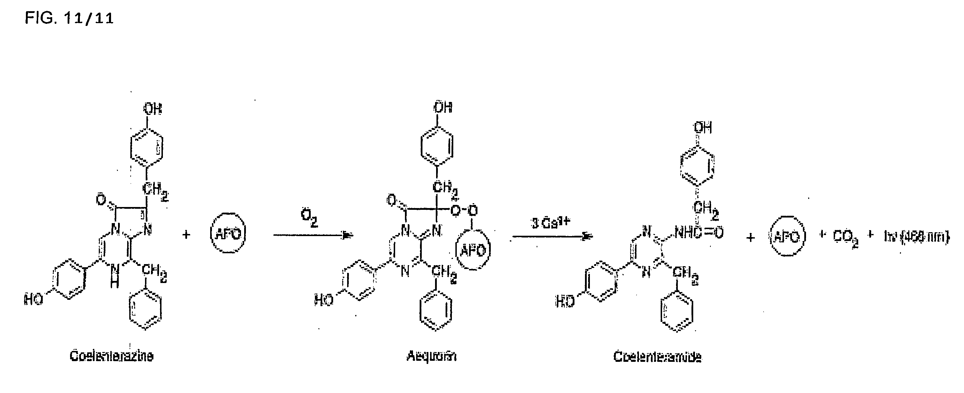

Apoaequorin is a protoprotein which, upon binding to coelenterazine, can emit photons in the presence Ca²⁺.
The Aequorin complex comprises a 22,514 MW Apoaequorin protein, molecular oxygen and the luminophore coelenterazine (Inouye et al., 1989;Johnson and Shimomura, 1978;Shimomura and Johnson, 1978). When three Ca²⁺ ions bind to this complex, coelenterazine is oxidized to coelenteramide, with a concomitant release of carbon dioxide and blue light (emission maximum -466 nm) (Figure 10).

Because of its Ca²⁺-dependent luminescence, the Aequorin complex has been extensively used as an intracellular Ca²⁺ indicator.

Aequorin reportedly does not disrupt cell functions or embryo development (Miller et al., 1994).

This photoprotein can be easily expressed in mammalian cells. It is utilized to monitor the cytosolic-free calcium concentration (Thomas and Delaville, 1991) (Sheu et al., 1993) (Stables et al., 2000).

Aequorin can also be easily targeted to specific organelles such as mitochondria (Brini et al., 1999) (Rizzuto et al., 1992) to monitor different aspects of calcium homeostasis.

The pharmaceutical industry takes widely advantage of the different properties of Aequorin, and particularly in High Throughput Screens (Detheux, 2000). The activation of a receptor coupled to the phospholipase C transduction pathway can be easily detected in presence of the photoprotein Aequorin, because of an instantaneous release of calcium from the endoplasmic reticulum.
WO0002045, Detheux et al. (EUROSCREEN S.A.) describes a high-throughput screening diagnostic and/or dosage method of an agonist and/or an antagonist for a calcium-coupled receptor (in mammalian cells) where Aequorin is used as marker for intracellular calcium changes upon receptor stimulation.

It has been previously shown that Aequorin can be functionally expressed in yeast and detected.

Nakajima-Shimada et al. (Nakajima-Shimada et al., 1991 b) describe the monitoring of intracellular calcium in Saccharomyces cerevisiae with an Apoaequorin cDNA expression system. Here, Aequorin was again used as a marker of intracellular calcium upon stress or glucose variations in the medium.

In contrast to mammalian signal transduction, there is no comparable Ca²⁺ release from the endoplasmic reticulum upon G protein-coupled receptor (GPCR) activation in yeast cells. The addition of α-factor to a cell (i.e. stimulation of the GPCR Ste2), from a basal level of approximately 100 nM, raises [Ca2+]i to a few hundred nanomolar in the cells, simultaneous with the induction of Ca2+ influx. When the cells are incubated with α- factor in a Ca2(+)-deficient medium, Ca2+ influx was greatly reduced, and the rise in [Ca2+]i was not detected (lida et al., 1990).

A limited number of reporter genes can be used in the yeast Saccharomyces cerevisiae and are not always appropriate. For screening purposes, the reporter product must be easy to detect and, for that reason, convenient reporter assays is required. The most commonly utilized reporter gene is LacZ which encodes the very big and stable enzyme β-Galactosidase.

Aequorin (22514 MW) is five times smaller than β-Galactosidase (116351 MW). Aequorin has a shorter half life than β-Galactosidase, therefore Aequorin is more suitable for growth/growth arrest quantification.

In summary, Aequorin is a very useful reporter gene, marker for intracellular Ca2+ variations in mammalian cells (Sheu et al., 1993).
Aequorin can be expressed functionally by the yeast S. cerevisiae (Nakajima-Shimada et al., 1991 a).
Aequorin is easily detected in a luminescent assay, in present of coelenterazin and Ca2+.

So far Aequorin has not been utilized as growth marker, being permanently expressed by the yeast cells.

In order to use Aequorin as a growth marker, the reporter gene must be permanently expressed (Aequorin is under the control of a constitutive yeast promoter). Then, each cell contains a comparable amount of reporter protein. If the assay is started with the same number of cells per well, at the end of the assay the Aequorin signal detected in each well will be proportional to the growth (i.e. to the new number of cells). Less stable than β-Galactosidase, Aequorin is more accurate to measure growth variations.

The present invention relates to a modified yeast cell containing an Aequorin encoding sequence under the control of a constitutive yeast promoter. In a special embodiment of the invention the Aequorin encoding sequence is SEQ ID NO. 1. In another special embodiment of the invention the constitutive yeast promoter is the Triose Phosphate Isomerase promoter. In another embodiment of the invention another heterologous protein; e.g. a heterologous kinase protein is in addition expressed in the modified yeast cell.

The present invention also relates to the use of the modified yeast cells, e.g. for screening of compounds.

Examples for yeast cells are Saccharomyces cerevisiae, Schizosaccharomyces pombe and candida albicans which can be modified by introducing heterologous sequences.

Method for identifying compounds that modulate heterologous protein-mediated growth or compounds that exhibit anti-fungal activity, wherein a modified yeast cell containing an Aequorin encoding sequence under the control of a constitutive yeast promoter is grown under conditions where the Aequorin protein is expressed. The modified yeast cell is incubated with a compound to be tested and the amount of growth in the presence of the compound is determined, preferably in comparison to the amount of growth in the absence of the compound or in the absence of the heterologous protein.

Aequorin succeed to be a better marker than β-Galactosidase to evaluate the number of cells per well in a 96-well plate, information expected in a high-throughput growth assay. In the same kind of assay, the toxicity of a compound can also be more easily detected with Aequorin than with β-Galactosidase, maybe because of the high stability of the second.

In another type of test, Aequorin could perfectly by utilized in assay adaptation for toxicity of an hyperactivated kinase.

Additionally, bacterial contamination sometimes occurs in the yeast assay and most of the contaminants physiologically express a β-Galactosidase. The contaminated cultures would give a very strong signal in presence of the β-Galactosidase substrate. The use of Aequorin eliminates the risk of false positives due to contamination.
- Figure 1:: Aequorin - open reading frame (SEQ ID NO. 1)
- Figure 2:: Restriction map of pYX132TPI-Aequorin
- Figure 3:: Restriction map of pYX132TPI-lacZ

- Figure 4:: Results of cell serial dilution - Linearity
- Figure 5:: Results of growth experiments - Yeast growth after 5 hours.
- Figure 6:: Results of growth experiments - Reporter detection after 5 hours
- Figure 7:: Results of growth experiments - Yeast growth after 24 hours
- Figure 8:: Results of growth experiments - Reporter detection after 24 hours
- Figure 9:: Results of toxicity detection in a kinase screen
- Figure 10:: Ca²⁺-dependent generation of luminescence by the aequorin complex, which contains apoaequorin (APO) and coelenterazine (Ohmiya and Hirano, 1996).

### Examples:

### Example 1: Materials and Methods

As a yeast strain W303 MAT a, far::hisG, sst2::URA3^{FOA}, fus1::HIS3 was used.

The yeast strain was transformed with the different plasmids according to the Lithium acetate method (Ito et al., 1983).

### Example 2: Construction of the Aequorin expression vectors

The full length cDNA aequorin gene was amplified by PCR using the chimerical mitochondrial aequorin mtAEQ (where the truncated N-terminus is fused to the human cytochrome C targeting signal (Rizzuto et al., 1992)) as a template. The 5' PCR primer contained the 50 first nucleotides of aequorin wild type sequence and an EcoRI (noted in bold type below) cloning site. The 3' primer did not contain any cloning site.
5' AQWT: 5'-CCG GAA TTC CGG ATG ACA AGC AAA CAA TAC TCA GTC AAG CTT ACA TCA GAC TTC GAC AAC CC (SEQ ID NO. 3)
3' AQWT: 5'-GGG CCT TAG GGG ACA GCT CCA CCG TAG AGC (SEQ ID NO. 4).

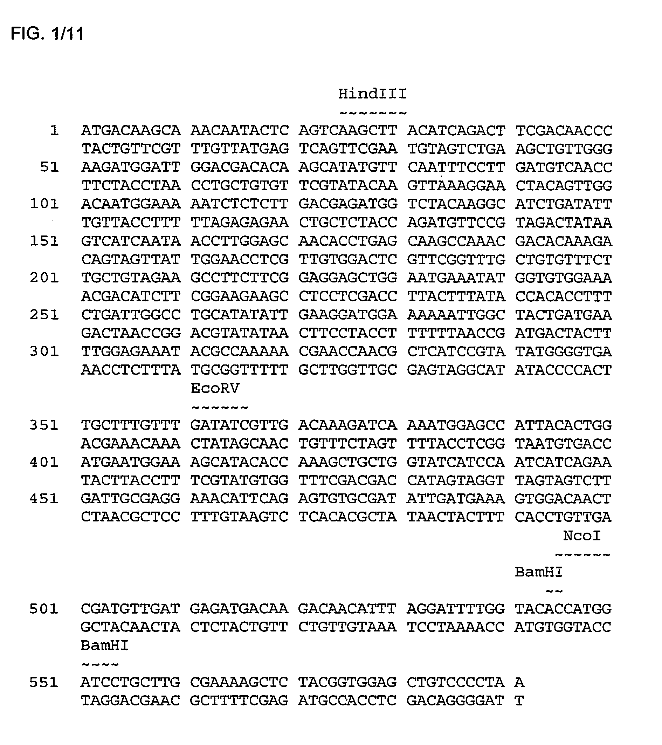

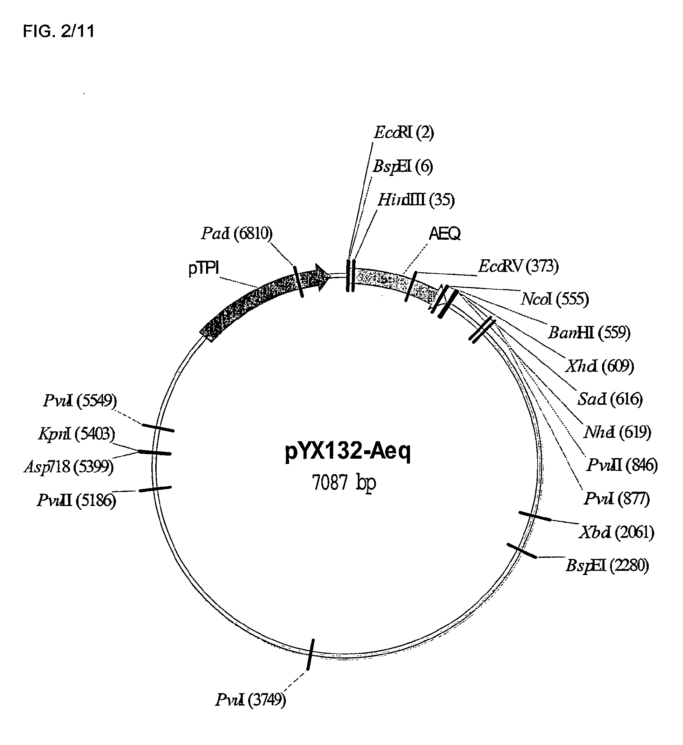

The full-length wild type Aequorin coding sequence (Figure 1) was then cloned in the commercially available yeast expression vector (Ingenius R&D) pYX132 TPI (Triose Phosphate Isomerase promoter, TRP1 marker, CEN), strong constitutive expression vector (FIG 2);

### Example 3: Construction of the β-Galactosidase expression vectors

To allow the comparison, LacZ was sub-cloned in the same way than aequorin, into the same expression vector (and without any fusion with the 5' sequence of a endogenous gene as is was often done to increase the expression level (King et al., 1990)).

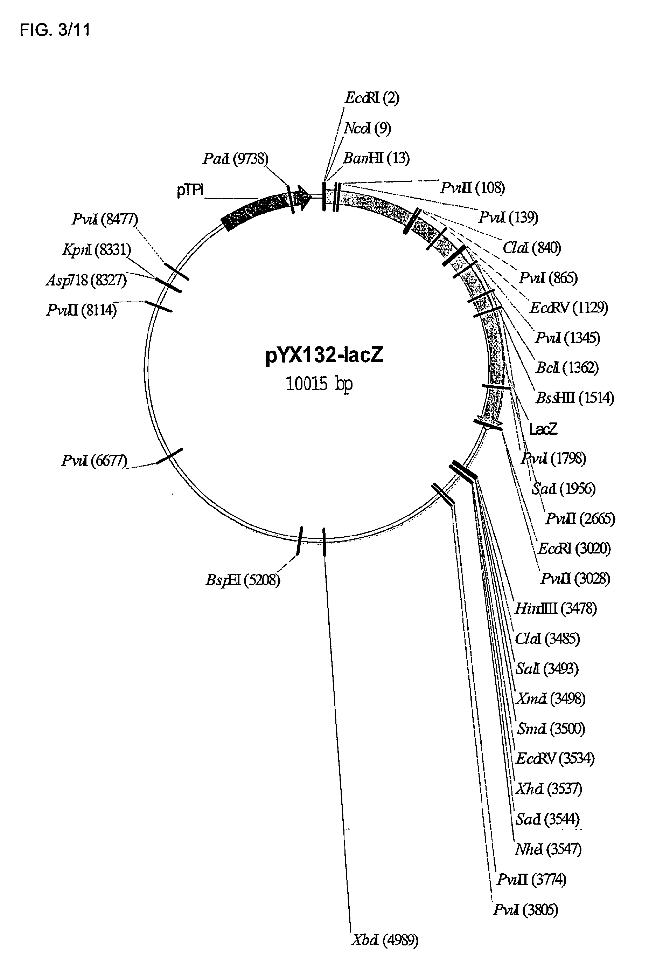

The full-length Escherichia coli β-Galactosidase gene sequence was cloned in the vector pYX132 TPI (Triose Phosphate Isomerase promoter,TRP1 marker, CEN), strong constitutive expression vector (FIG 3);

### Example 4: Aequorin detection

The cells are distributed and/or grown in a white 96-well plate, in a volume of 100µl. 30 minutes before the measurement time point, 10µl of a 5µM coelenterazine (Molecular Probes) solution is distributed in each well (to obtain a 0,5µM final concentration) to load the cells.

The plate is then incubated at 30°C for the last 30 minutes.

Aequorin detection is made in a luminometer with injecting system (Luminoskan, Labsystems). For each well, immediately after injection of a 10mM CaCl2 solution (1 M CaCl2 diluted in lysis buffer Y-PER from Pierce), the luminescent signal is integrated for 15 seconds.

To avoid the intermediate step of loading, it is possible to introduce coelenterazine in the medium at the beginning of the assay: coelenterazine is added at a concentration of 0.5µM.

### Example 5: β-Galactosidase detection

The cells are distributed and/or grown in a white 96-well plate, in a volume of 100µl.

At the measurement time point, each well receives 100µl of a β-Galactosidase detection mix (Gal-screen, Tropix).

The plate is incubated for one hour at 28°C.

β-Galactosidase signal is read in a luminometer ; the luminescent signal is integrated for 0.5 seconds.

### Example 6: Growth marker: aequorin compared to lacZ

### Example 6.1: Linearity

The pYX132TPI expression plasmid was chosen because it combines a reproducible number of copies per cell (centromeric replication origin) with a constitutive strong expression level (Triose Phosphate Isomerase promoter (Alber and Kawasaki, 1982)).

Three colonies of the yeast strain transformed with either pYX132TPI-AEQ or pYX132TPI-LacZ plasmids were grown in the appropiate medium (SC Glucose-Trp) to stationary phase and then serially diluted in the same medium (with 0.5µM coelenterazin for the aequorin strain): from 10⁶ to 10⁴ cells per well. The plate was then shaken at 30°C, 700 r.p.m., for 1.5 hours to re-initiate the exponential growth.

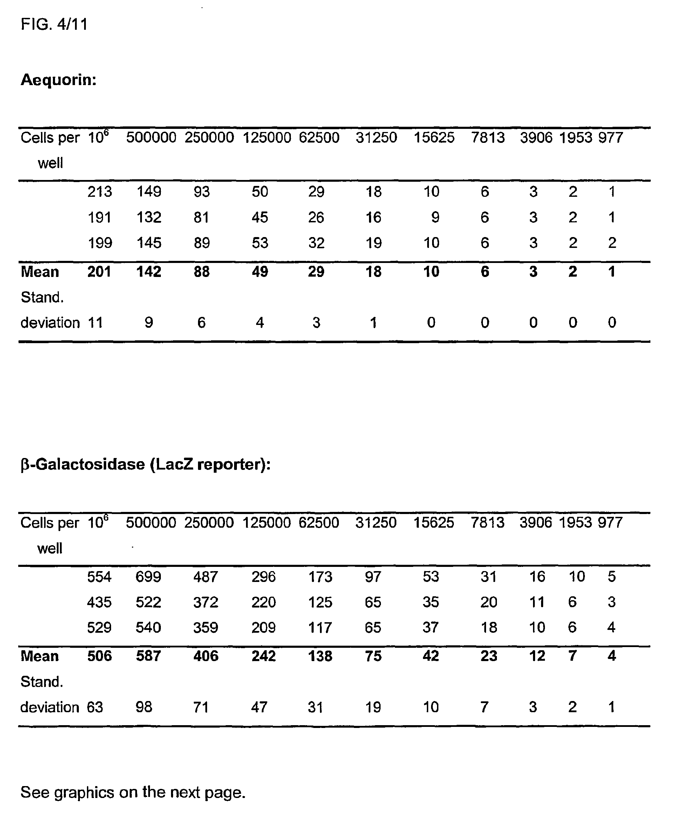

The Figure 4 shows the difference of linearity obtained with the two reporter genes: aequorin would be suitable until 3 to 4.10⁵ cells per well whereas the lacZ signal reaches very rapidly a plateau (linearity only until 10⁵).

### Example 6.2: Stability upon toxicity or growth arrest

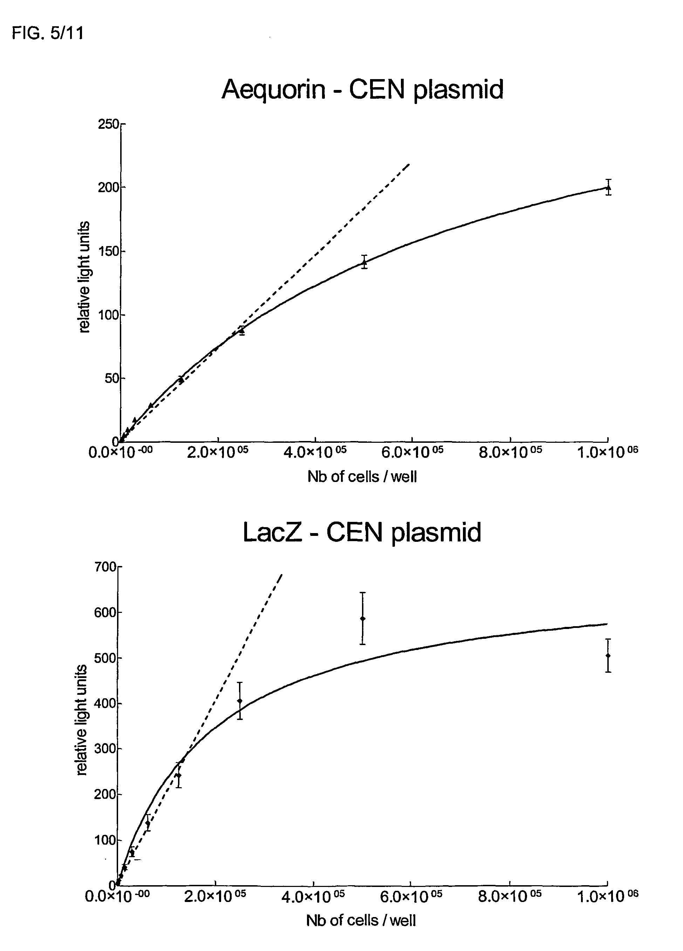

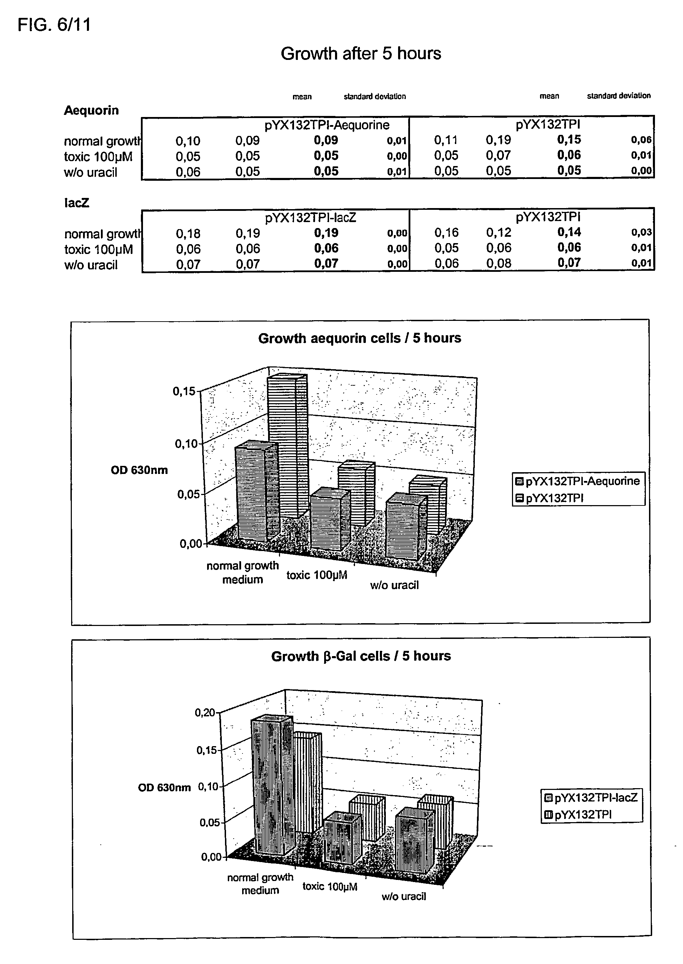

Two colonies of the yeast strain transformed with either pYX132TPI-AEQ or pYX132TPI-LacZ or empty pYX132TPI plasmids were grown in the appropiate medium (SC Glucose -Trp) to stationary phase and then diluted in three different media:
- SC Glucose -Trp (normal growth medium);
- SC Glucose -Trp containing 100µM of a toxic compound (Ziprasidone, a potassium channel blocker that shows high toxic effects on S. cerevisiae);
- SC Glucose -Ura (the strain will starve because it needs uracile to grow).
   The results are shown on Figures 5 to 8.
   After 5 hours of treatment (FIG 5 & FIG 6), the toxic effect was well detectable in a optical density (OD) measurement (OD₆₃₀=0.05 to 0.07 in stead of 0.09 to 0.19) as well as with both reporter genes.
- Aequorin showed, for the cells in presence of Ziprasidone, a signal equivalent to the starving cells (10 and 16 r.l.u., respectively) while the normal growth would give a aequorin signal of 138. In other words, aequorin showed a 10 fold difference between toxic treatment and normal growth.
- β-Galactosidase reflected also the toxicity but in a much lower contrast: the normal growth woul dgive a signal of 276 r.l.u. while the Ziprasidone treated cells produce 116 r.l.u. (i.e. only half of the normal signal), and the starving cells give a signal of 64 r.l.u..

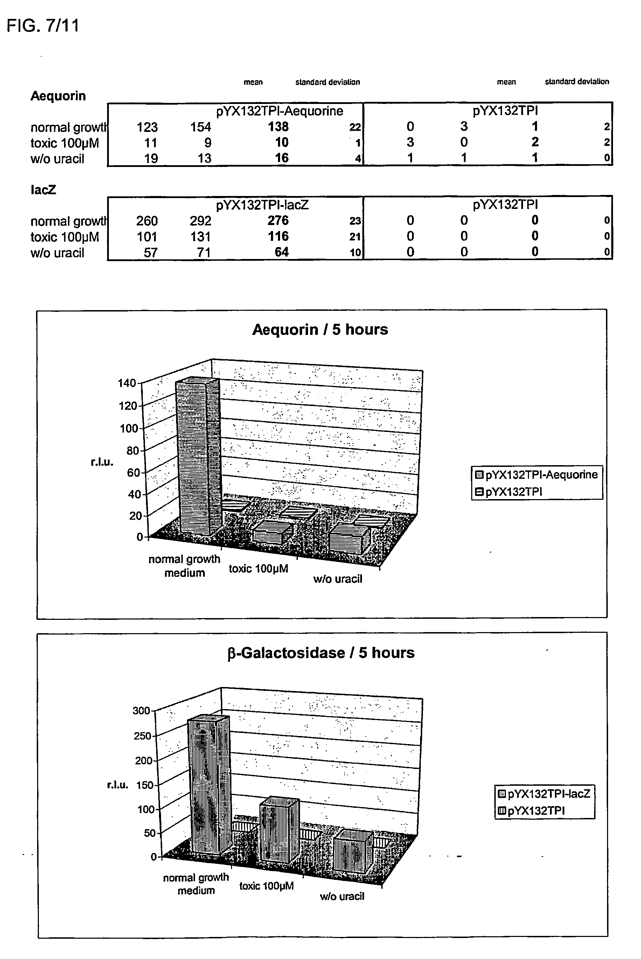

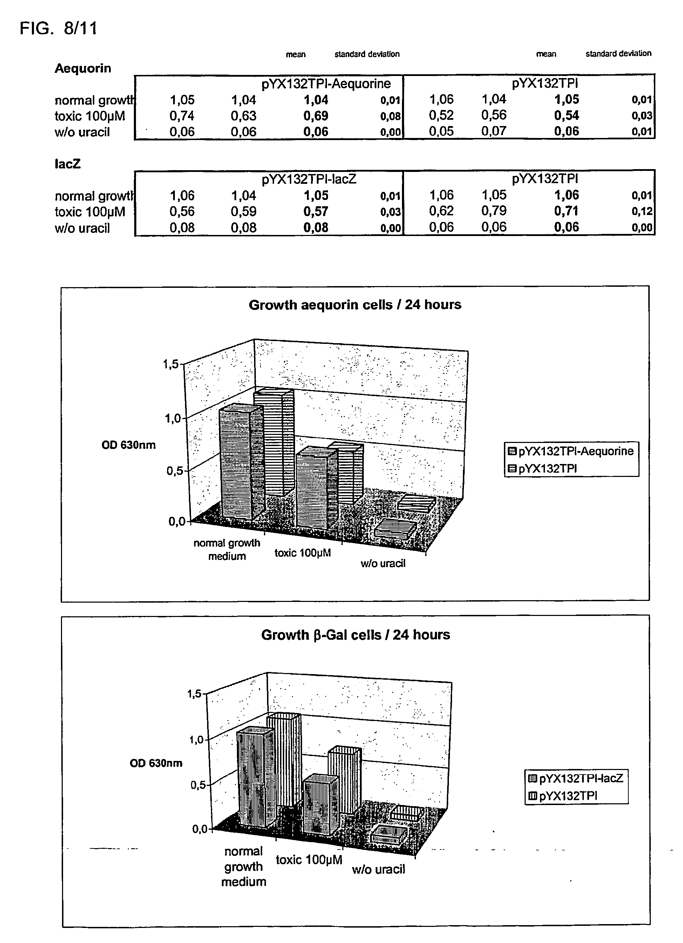

After 24 hours treatment (FIG 7 & FIG 8), the cells in the medium lacking uracile did not grow, they could recover from the toxicity of Ziprasidone and grow up to OD₆₃₀=0.5-0.7, while the cells in the appropriate medium grew to saturation (i.e. to OD₆₃₀ over 1). Again this effect was better reflected by aequorin:
- with aequorin, after toxic treatment the signal was 50 r.l.u. and after normal growth 175 r.l.u.; that is to say a difference of 3.5 fold;
- the β-Galactosidase numbers for treated or non treated cells were not significantly different (respectively 1923 and 2275 r.l.u.).

This experiment shows that β-Galactosidase is not suitable, in a short assay (5 hours), to show a suffering of the yeast cell. The sensitivity is much higher with aequorin. This result might be due to the high stability of the protein β-Galactosidase.

In a longer assay (24 hours), the difference of sensitivity of the two reporters appears to be even more significant.

### Example 7: Screening for inhibitors of a human kinase

A human kinase, potentially therapeutic target, is modified to become constitutively active. The activated kinase is toxic for the yeast and induces cell death. Both kinase and Aequorin are under the control of a promotor induced only in presence of galactose.
The cells are grown in SC Glucose, rinced, and then diluted to 10⁶ cells/ml in SC/Glucose or SC/Galactose. After 30 hours incubation at 30°C, coelenterazine is added and the Aequorin signal measured after injection of Ca2+, accordingly to the Aequorin detection protocol.

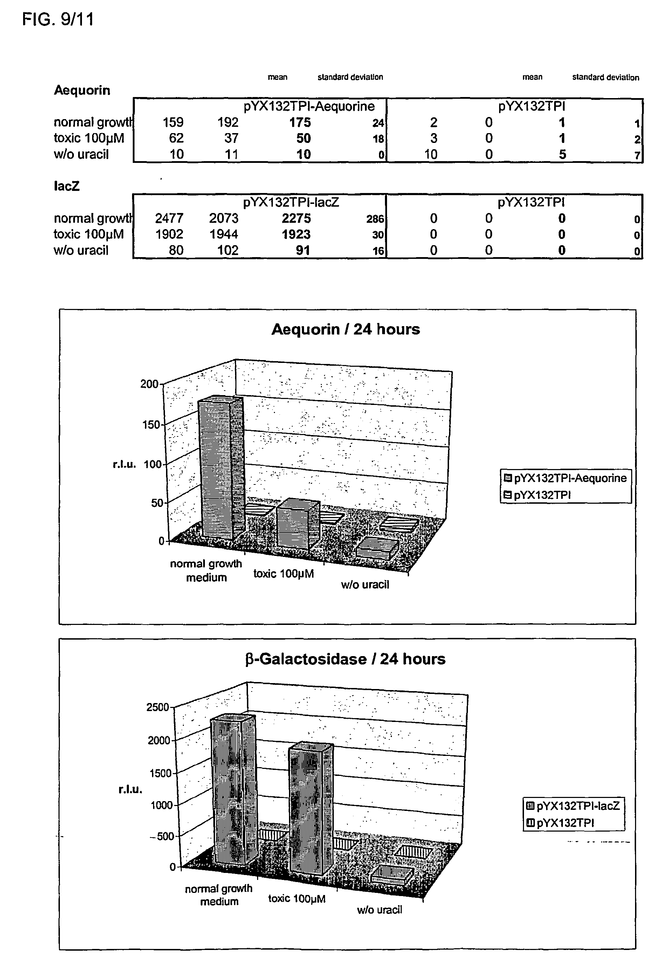

The Figure 9 shows that in glucose medium, there is a very slide expression of Aequorin but comparable in the two strains.
In galactose medium (both Aequorin and the active kinase are expressed) the Aequorin signal is very high (7457 relative light units) in absence of any toxicity, but disappears completely (0,5 r.l.u.) when the active kinase is expressed.

This example is a preliminary test for set up of a kinase inhibitor screening: the compound which can rescue cell death is selected for its potential inhibitory activity on the kinase.

### Reference List:

Alber T and Kawasaki G (1982) Nucleotide Sequence of the Triose Phosphate Isomerase Gene of Saccharomyces Cerevisiae. J Mol Appl Genet 1: pp 419-434.
Brini M, Pinton P, Pozzan T and Rizzuto R (1999) Targeted Recombinant Aequorins: Tools for Monitoring [Ca2+] in the Various Compartments of a Living Cell. Microsc Res Tech 46: pp 380-389.
Detheux M (2000) Orphan Receptors: the Quest for New Drug Targets. Innovations in Pharmaceutical Technology 00: pp 27-34.
lida H, Yagawa Y and Anraku Y (1990) Essential Role for Induced Ca2+ Influx Followed by [Ca2+]i Rise in Maintaining Viability of Yeast Cells Late in the Mating Pheromone Response Pathway. A Study of [Ca2+]i in Single Saccharomyces Cerevisiae Cells With Imaging of Fura-2. J Biol Chem 265: pp 13391-13399.
Inouye S, Aoyama S, Miyata T, Tsuji F I and Sakaki Y (1989) Overexpression and Purification of the Recombinant Ca2+-Binding Protein, Apoaequorin. J Biochem (Tokyo) 105: pp 473-477.
Ito H, Fukuda Y, Murata K and Kimura A (1983) Transformation of Intact Yeast Cells Treated With Alkali Cations. J Bacteriol 153: pp 163-168.
Johnson FH and Shimomura O (1978) Bioluminescence and Chemiluminescence: Introduction to the Bioluminescence of Medusae, With Special Reference to the Photoprotein Aequorin. Methods Enzymol 57: pp 1-653.
King K, Dohlman H G, Thorner J, Caron M G and Lefkowitz R J (1990) Control of Yeast Mating Signal Transduction by a Mammalian □2-Adrenergic Receptor and Gs Alpha Subunit [Published Erratum Appears in Science 1991 Jan 11;251(4990):144]. Science 250: pp 121-123.
Miller AL, Karplus E and Jaffe L F (1994) Imaging [Ca2+]i With Aequorin Using a Photon Imaging Detector. Methods Cell Biol 40: pp 305-338.
Nakajima-Shimada J, lida H, Tsuji F I and Anraku Y (1991 a) Galactose-Dependent Expression of the Recombinant Ca2(+)-Binding Photoprotein Aequorin in Yeast. Biochem Biophys Res Commun 174: pp 115-122.
Nakajima-Shimada J, lida H, Tsuji F I and Anraku Y (1991 b) Monitoring of Intracellular Calcium in Saccharomyces Cerevisiae With an Apoaequorin CDNA Expression System. Proc Natl Acad Sci U S A 88: pp 6878-6882.
Ohmiya Y and Hirano T (1996) Shining the Light: the Mechanism of the Bioluminescence Reaction of Calcium-Binding Photoproteins. Chem Biol 3: pp 337-347.
Rizzuto R, Simpson A W, Brini M and Pozzan T (1992) Rapid Changes of Mitochondrial Ca2+ Revealed by Specifically Targeted Recombinant Aequorin [Published Erratum Appears in Nature 1992 Dec 24- 31;360(6406):768]. Nature 358: pp 325-327.
Sheu YA, Kricka L J and Pritchett D B (1993) Measurement of Intracellular Calcium Using Bioluminescent Aequorin Expressed in Human Cells. Anal Biochem 209: pp 343-347.
Shimomura O and Johnson F H (1978) Peroxidized Coelenterazine, the Active Group in the Photoprotein Aequorin. Proc Natl Acad Sci U S A 75: pp 2611-2615.
Stables J, Mattheakis L C, Chang R and Rees S (2000) Recombinant Aequorin As Reporter of Changes in Intracellular Calcium in Mammalian Cells. Methods Enzymol 327: pp 456-471.
Thomas AP and Delaville F (1991) The Use of Fluorescent Indicators for measurements of cytosolic-free calcium concentration in cell populations and single cells., in Cellular Calcium: A Practical Approach (McCormack JG and Cobbold PH eds) pp 1-54.

## Claims

1. A method for determining cell growth or number of cells, the method comprising the steps of
a) growing a modified yeast cell containing an Aequorin-encoding sequence under the control of a constitutive yeast promoter under conditions, wherein the Aequorin protein is permanently and equally expressed in each cell,
b) detecting an Aequorin signal, and
c) determining cell growth or number of cells, wherein the Aequorin signal is indicative of the cell growth or number of cells.

2. A method for identifying compounds that modulate heterologous protein-mediated growth or compounds that exhibit toxicity, the method comprising the steps of:
a) growing a modified yeast cell containing an Aequorin-encoding sequence under the control of a constitutive yeast promoter under conditions wherein the Aequorin protein is permanently and equally expressed in each cell,
b) incubating the modified cell with a compound to be tested,
c) detecting an Aequorin signal, and
d) determining the change in growth or survival in the presence of the compound,
wherein the Aequorin signal is indicative the change in growth or survival.

3. The method of claim 2, wherein the change in growth or survival is determined by comparing growth or survival in the presence and absence of the compound to be tested.

4. The method of claim 2, wherein the change in growth or survival is determined by comparing growth or survival in the presence and absence of the heterologous protein.

5. The method of claim 4, wherein the heterologous protein is a heterologous kinase protein.

6. The method of any of claims 1 to 5, wherein the promoter is triose phosphate isomerase promoter.

7. The method of any of claims 1 to 6, wherein the Aequorin-encoding sequence is SEQ ID NO: 1.

8. The method of any of claims 1 to 7, wherein the yeast cell is Saccharomyces cerevisiae, Schizosaccharomyces pombe or Candida albicans.

9. The method of any of claims 1 to 8, wherein the Aequorin signal is a luminescence signal.

## Patentansprüche

1. Verfahren zur Bestimmung des Zellwachstums oder der Zellzahl, wobei das Verfahren die folgenden Schritte umfaßt:
a) Züchten einer modifizierten Hefezelle, die eine Aequorin-Codiersequenz unter der Kontrolle eines konstitutiven Hefepromoters enthält, unter Bedingungen, bei denen das Aequorinprotein permanent und gleichmäßig in jeder Zelle exprimiert wird,
b) Nachweisen eines Aequorinsignals, und
c) Bestimmen des Zellwachstums oder der Zellzahl, wobei das Aequorinsignal das Zellwachstum oder die Zellzahl angibt.

2. Verfahren zum Identifizieren von Verbindungen, die das von einem heterologen Protein vermittelte Wachstum modulieren oder von Verbindungen, die toxisch wirken, wobei das Verfahren die folgenden Schritte umfaßt:
a) Züchten einer modifizierten Hefezelle, die eine Aequorin-Codiersequenz unter der Kontrolle eines konstitutiven Hefepromoters enthält, unter Bedingungen, bei denen das Aequorinprotein permanent und gleichmäßig in jeder Zelle exprimiert wird,
b) Inkubieren der modifizierten Zelle mit einer Testverbindung,
c) Nachweisen eines Aequorinsignals, und
d) Bestimmen der Veränderung bezüglich Wachstum oder Überleben in Gegenwart der Verbindung, wobei das Aequorinsignal das Zellwachstum oder die Zellzahl angibt.

3. Verfahren nach Anspruch 2, wobei die Veränderung bezüglich Wachstum oder Überleben **dadurch** bestimmt wird, daß man das Wachstum oder Überleben in Gegenwart bzw. Abwesenheit der Testverbindung bestimmt.

4. Verfahren nach Anspruch 2, wobei die Veränderung bezüglich Wachstum oder Überleben **dadurch** bestimmt wird, daß man das Wachstum oder Überleben in Gegenwart bzw. Abwesenheit des heterologen Proteins bestimmt.

5. Verfahren nach Anspruch 4, wobei es sich bei dem heterologen Protein um ein heterologes Kinaseprotein handelt.

6. Verfahren nach einem der Ansprüche 1 bis 5, wobei es sich bei dem Promoter um den Triosephosphatisomerase-Promoter handelt.

7. Verfahren nach einem der Ansprüche 1 bis 6, wobei es sich bei der Aequorin-Codiersequenz um SEQ ID NO:1 handelt.

8. Verfahren nach einem der Ansprüche 1 bis 7, wobei es sich bei der Hefezelle um Saccharomyces cerevisiae, Schizosaccharomyces pombe oder Candida albicans handelt.

9. Verfahren nach einem der Ansprüche 1 bis 8, wobei es sich bei dem Aequorinsignal um ein Lumineszenzsignal handelt.

## Revendications

1. Méthode permettant de déterminer la croissance cellulaire ou le nombre de cellules, la méthode comprenant les étapes consistant à :
a) cultiver une cellule de levure modifiée contenant une séquence codant pour l'aequorine sous le contrôle d'un promoteur constitutif de levure dans des conditions dans lesquelles la protéine aequorine est exprimée de façon permanente et égale dans chaque cellule,
b) détecter un signal aequorine, et
c) déterminer la croissance cellulaire ou le nombre de cellules, le signal aequorine étant indicateur de la croissance cellulaire ou du nombre de cellules.

2. Méthode permettant d'identifier des composés qui modulent la croissance médiée par une protéine hétérologue ou des composés qui présentent une toxicité, la méthode comprenant les étapes consistant à :
a) cultiver une cellule de levure modifiée contenant une séquence codant pour l'aequorine sous le contrôle d'un promoteur constitutif de levure dans des conditions dans lesquelles la protéine aequorine est exprimée de façon permanente et égale dans chaque cellule,
b) incuber la cellule modifiée avec un composé à tester,
c) détecter un signal aequorine, et
d) déterminer le changement de croissance ou de survie en présence du composé, le signal aequorine étant indicateur du changement de croissance ou de survie.

3. Méthode selon la revendication 2, selon laquelle le changement de croissance ou de survie est déterminé en comparant la croissance ou la survie en présence et en l'absence du composé à tester.

4. Méthode selon la revendication 2, selon laquelle le changement de croissance ou de survie est déterminé en comparant la croissance ou la survie en présence et en l'absence de la protéine hétérologue.

5. Méthode selon la revendication 4, selon laquelle la protéine hétérologue est une protéine kinase hétérologue.

6. Méthode selon l'une quelconque des revendications 1 à 5, selon laquelle le promoteur est le promoteur de la triose phosphate isomérase.

7. Méthode selon l'une quelconque des revendications 1 à 6, selon laquelle la séquence codant pour l'aequorine est SEQ ID NO : 1.

8. Méthode selon l'une quelconque des revendications 1 à 7, selon laquelle la cellule de levure est Saccharomyces cerevisiae, Schizosaccharomyces pombe ou Candida albicans.

9. Méthode selon l'une quelconque des revendications 1 à 8, selon laquelle le signal aequorine est un signal de luminescence.
